# EUROPÄISCHE PATENTSCHRIFT

(11) **EP 2 111 814 B1**
(45) Veröffentlichungstag und Bekanntmachung des Hinweises auf die Patenterteilung: **01.08.2012**
(21) Anmeldenummer: 08155032.9
(22) Anmeldetag: 23.04.2008
(51) Int. Cl.: A61B 6/03, A61B 19/00

(54) **Verfahren zum Registrieren eines mit fächerförmigen Abbildungsstrahlen generierten 2D-Bilddatensatzes im Bereich der Medizin und dazugehöriges Computerprogrammprodukt sowie Verfahren und System zur automatischen Registrierung eines Körpers basierend auf 2D-Bilddaten zum Einsatz in medizinischen Navigationssystemen**
Method of registering a 2D image data recorded with fan-shaped mapping rays for medicine and associated computer program product and method and system to automatically register a body based on 2D image data for use in medical navigation systems
Procédé d'enregistrement d'un ensemble de données d'image en 2D généré par des rayons de reproduction en forme d'éventail dans le domaine de la médecine et produit de programme informatique correspondant ainsi que procédé et système d'enregistrement automatique d'un corps basé sur des données d'image en 2D pour l'utilisation avec des systèmes de navigation médicaux

(43) Veröffentlichungstag der Anmeldung: 28.10.2009
(73) Patentinhaber: BrainLAB AG, 85622 Feldkirchen (DE)
(72) Erfinder: Edlauer, Martin, 80809 München (DE); Mezger, Uli, 81543 München (DE); Essenreiter, Robert, 81737 München (DE); Weiser, Manfred, 81825 München (DE)
(74) Vertreter: Schwabe - Sandmair - Marx

(56) Entgegenhaltungen:
- EP-A- 1 192 913
- WO-A2-2007/092841
- DE-A1- 10 334 163
- US-A1- 2004 127 788

## Beschreibung

### Gebiet der Erfindung

Die Erfindung bezieht sich auf ein Verfahren zur Registrierung eines mit fächerförmigen Abbildungsstrahlen generierten 2D-Bilddatensatzes im Bereich der Medizin. Des Weiteren bezieht sich die Erfindung auf ein Verfahren zum automatischen Registrieren eines Körpers basierend auf 2D-Bilddaten, die zum Einsatz in medizinischen Navigationssystemen geeignet sind, in dem die 2D-Bilddaten mittels fächerförmigen Abbildungsstrahlen generiert worden sind und mittels des erfindungsgemäßen Verfahrens registriert werden. Die Erfindung bezieht sich weiter auf ein Computerprogrammprodukt mit einem Programmcode zum Ausführen des erfindungsgemäßen Verfahrens zum Registrieren. Schließlich bezieht sich die Erfindung auf ein System zur automatischen Registrierung eines Körpers basierend auf 2D-Bilddaten zum Einsatz in medizinischen Navigationssystemen. Bei dem Körper handelt es sich zum Beispiel um den Körper eines Patienten oder um Teile davon, zum Beispiel Gliedmaßen oder einen Torsos.

### Stand der Technik

Im Stand der Technik sind verschiedenste Verfahren zur Registrierung eines Körpers basierend auf verschiedenen Bilddatensätzen bereits bekannt. Dabei bedeutet Registrieren eines Körpers, dass bei einer Abbildung dieses Körpers die exakte räumliche Position des Körpers ermittelt wird. Dies ermöglicht eine Zuordnung von räumlichen Positionen zu Bilddaten. Diese Zuordnung ermöglicht z.B. bei Navigations-unterstützten Operationen, chirurgische Instrumente genau in solche Positionen zu bringen, die der Operateur vorher anhand von Aufnahmen festgelegt hat.

Bei den bekannten Registrierungsverfahren wird zwischen manuellen Verfahren und automatischen Registrierungsverfahren unterschieden. Bei den manuellen Verfahren kommt z.B. ein Pointer zum Einsatz, mit dem auf bestimmte Positionen eines abzubildenden Körpers gedeutet wird. Dabei wird die Position des Pointers im Raum bestimmt. Zum anderen wird händisch in einem vorliegenden Bilddatensatz die Position zusätzlich angegeben, an der sich der Pointer befindet. Auf diese Weise kann ein zu registrierender Körper mit dem Pointer abgetastet werden, und Schritt für Schritt wird durch ein Punkt-Mapping eine Zuordnung zwischen räumlichen Positionen und Bilddatenpunkten erreicht.

Bei automatischen Registrierverfahren kommt beispielsweise ein sog. Kalibrierphantom zum Einsatz. Während der Aufnahme eines Bilddatcnsatzes, der für Navigationszwecke geeignet ist, wird zusätzlich zu dem Körper, an dem eine Operation durchgeführt werden soll, ein Kalibrierreferenzkörper gleichzeitig mit aufgenommen. Die Position dieses Referenzkörpers im Raum ist aber bekannt und wird z.B. mittels eines Kamerasystems aufgezeichnet. Hierbei kommen normalerweise sog. Markereinrichtungen zum Einsatz, die mit einem Kamerasystem erkannt werden. Ein solches Markersystem befindet sich dabei am Referenzkörper und ein weiteres, das gleichzeitig von dem Kamerasystem erkannt wird, befindet sich in vorbekannter relativer Position zum Aufnahmegerät, meist am Aufnahmegerät selbst. Auf diese Weise kann eine automatische Zuordnung von Bilddaten zu räumlichen Koordinaten erfolgen.

Obwohl bereits eine Vielzahl von unterschiedlichen Registrierverfahren bekannt ist, sind diese Registrierverfahren zur Zeit auf bestimmte Arten von Bilddatensätzen beschränkt. Bilddatensätze, basierend auf denen eine Registrierung vorgenommen werden kann, sind derzeit z.B. volumetrische Bilddatensätze, d.h. 3D-Datensätze, wie sie z.B. mittels Computertomographen und magnetischen Resonanztomographen erzeugt werden. Des Weiteren existieren automatische Registrierverfahren für bestimmte 2D-Bilddatensätze, bei denen ein kegeförmiger Aufnahmestrahl verwendet wird. Dies ist z.B. bei Aufnahmen mittels Röntgengeräten vom C-Bogen-Typ und bei Ultraschallaufnahmen der Fall.

Fluoroskopische Röntgenaufnahmen, bei denen die räumliche Position des aufgenommenen Körpers bekannt ist, werden heute ebenfalls dazu verwendet, die räumlichen Positionen von Punkten in 3D CT-Aufnahmen zu bestimmen (sog. CT-Fluoro Matching).

DE 103 34 163 A1 offenbart Lokalisatoren zur Erstellung von Übersichtsaufnahmen für Computertomographen, die an einem stereotaktischen Ring befestigt sind und die die genaue Bestimmung von Positionen im Gehirn ermöglichen.

US 2004/0127788 A1 offenbart ein Verfahren und eine Vorrichtung zur bildunterstützten Navigation, das die Registrierung von Bilddatensätzen und die anschließende Navigation zur Unterstützung basierend auf den registrierten Bilddatensätzen ermöglicht. Dazu werden verschiedene Transformation und unterschiedliche Arten von Markern verwendet.

WO 2007/092841 A2 offenbart Vorrichtungen und chirurgische Werkzeuge zum Ersatz von Gelenken. Insbesondere kann ein Anwender damit den Mittelpunkt des Sprunggelenks und den Mittelpunkt des Hüftgelenks für Knieoperationen mittels eines CT-Scout-Scans ermitteln.

Aus dem Stand der Technik ist hingegen kein Registrierungsverlahren bekannt, das Bilder verwendet, die mit fächerförmigen Abbildungsstrahlen erzeugt worden sind. Aus mehreren fächerförmigen Abbildungsstrahlen, die gegeneinander räumlich versetzt sind (z.B. parallel verschoben), wird dabei ein 2D-Bilddatensatz zusammengesetzt.

Fächerförmige Abbildungsstrahlen zeichnen sich dadurch aus, dass der Abbildungsstrahl in einer bestimmten Vorzugsrichtung weit aufgefächert ist (Auffächerungsrichtung), wohingegen der Abbildungsstrahl in einer Richtung orthogonal zur Aufficherungsrichtung eine extrem geringe Aufweitung besitzt. Das Verhältnis entspricht in etwa 1m (Abstand Detektor - Quelle) zu 1mm (ungefähre Dicke einer Detektorreihe in z-Richtung).

Es handelt sich bei einem fächerförmigen Abbildungsstrahl somit um einen speziellen gerichteten Strahl, der sich aus einer Vielzahl von Einzelstrahlen zusammensetzt und im Wesentlichen in einer Ebene liegt. Die geometrischen Eigenschaften des Fächerstrahles lassen sich somit durch einen Winkel in der Ebene, der die Auffächerung des Fächerstrahles beschreibt, näherungsweise gut charakterisieren. Solche fächerförmigen Abbildungsstrahlen werden z.B. im Vorfeld einer eigentlichen CT-Aufnahme mit einem Computertomographen erzeugt. Diese sog. Topogramme oder Scoutviews werden normalerweise dazu verwendet, den Scan-Bereich der Hauptaufnalme festzulegen. Während bei der eigentlichen CT-Aufnahme die Strahlungsquelle um den zu durchleuchtenden Körper herum rotiert und einzelne fokussierte Strahlen ohne nennenswerte Aufweitung ausgesendet werden, ist bei 2D CT-Scan-Daten die Strahlungsquelle bezogen auf den Körper räumlich fix, und es wird ein fächerförmiger Abbildungsstrahl ausgesendet. Der zu durchleuchtende Körper wird dann unter der Strahlungsquelle hindurch geschoben und mehrere fächerförmige Aufnahmen werden gemacht, aus denen dann ein 2D-Bilddatensatz zusammengesetzt wird. Natürlich ist es auch möglich, dass nicht der Körper, sondern die Strahlungsquelle bewegt wird. Entscheidend ist die relative Verschiebung von Körper und Strahlungsquelle zueinander.

Es ist die Aufgabe der vorliegenden Erfindung, ein verbessertes Verfahren zum automatischen Registrieren eines Körpers basierend auf 2D-Bilddaten, die zum Einsatz von medizinischen Navigationssystemen geeignet sind, und ein System zur automatischen Registrierung eines Körpers basierend auf 2D-Bilddaten zum Einsatz in medizinischen Navigationssystemen bereitzustellen.

### Beschreibung der Ereindung

Die Aufgabe wird gelöst durch den Gegenstand der unabhängigen Ansprüche 1 und 10.

Die Unteransprüche sind auf bevorzugte Ausführungsformen der Erfindung gerichtet.

Den Kern des erfindungsgemäßen Verfahrens und des erfindungsgemäßen Systems bildet ein neuartiges Verfahren zum Registrieren eines mit fächerförmigen Abbildungsstrahlen generierten 2D-Bilddatensatzes im Bereich der Medizin. Insbesondere wird es aufgrund dieses Verfahrens möglich, die eben geschilderten 2D CT-Sean-Daten für Registrierungs- und anschließende Navigationszwecke zu verwenden. Dies hat den Vorteil, dass für die Registrierung und Navigation Bilder mit großer Detailschärfe verwendet werden können, was eine genauere Navigation ermöglicht.

Ein weiterer Vorteil ist, dass für Navigationszwecke Bilder eingesetzt werden können, die sowieso im Vorfeld einer CT-Aufnahme erstellt werden. Dies senkt die Strahlenbelastung für einen Patienten. Des Weiteren ist die Strahlungsbelastung durch 2D CT-Scan-Aufnahmen geringer als bei einer herkömmlichen fluoroskopischen Röntgenaufnahme.

Das erfindungsgemäße Verfahren und System hat überdies den Vorteil, dass keinerlei spezifische Informationen über das verwendete Abbildungsgerät bzw. über dessen Transferfunktion notwendig sind. Es ist somit insbesondere möglich, das erfindungsgemäße Verfahren herstellerunabhängig einzusetzen.

Ein erfindungsgemäßes Verfahren zum Registrieren eines mit fächerförmigen Abbildungsstrahlen generierten 2D-Bilddatensatzes im Bereich der Medizin weist zunächst einen Bearbeitungsschritt auf, der 2D-Bilddaten basierend auf einer Raumtransformationsfunktion, die eine räumliche Relativposition zwischen Punkten, die mit einem fächerförmigen Abbildungsstrahl abgebildet worden sind, und dem zur Abbildung verwendeten Abbildungsgerät beschreibt. Unter einem fächerförmigen Abbildungsstrahl wird ein Abbildungsstrahl verstanden, der von einer Strahlungsquelle ausgesendet wird und in einer Dimension eine starke Aufweitung in Fächerform erfährt. In einer Richtung orthonogonal zu dieser Auffächerungsrichtung ist die Aufweitung des Strahles vernachlässigbar gering. Insgesamt liegen somit alle fächerförmigen Abbildungsstrahlen im Wesentlichen in ein und derselben Ebene. Die Detektion eines fächerförmigen Abbildungsstrahles ist somit mittels eines Detektors möglich, der linear z.B. in Form eines Zeitendetektors ausgestaltet ist. Aus mehreren räumlich gegencinander z.B. parallel verschobenen fächerförmigen Abbildungsstrahlen kann auf diese Weise ein 2D-Bilddatensatz zusammengesetzt werden.

Bei der Raumtransformationsfunktion handelt es sich bevorzugt um eine Funktion, die ausschließlich die Lage von Punkten im Raum berücksichtigt, nicht hingegen bestimmte Abbildungseigenschaften eines Abbildungsgerätes. In einem bevorzugten Ausführungsbeispiel beschreibt die Raumtransformationsfunktion eine räumliche Relativposition zwischen Punkten, die mit einem fächerförmigen Abbildungsstrahl abgebildet worden sind und dem zur Abbildung verwendeten Abbildungsgerät. Die Raumtransformationsfunktion erlaubt eine direkte (d.h. einstufige) oder indirekte (d.h. mehrstufige) Transformation in ein Bezugssystem des Abbildungsgerätes. Wird auf mehrere abzubildende bzw. bereits abgebildete Punkte Bezug genommen, so können die Positionsdaten von diesen Punkten in absoluten Werten bezüglich des Koordinatensystems des Abbildungsgerätes beschrieben werden. Es ist alternativ auch möglich, die relative Lage von abgebildeten Punkten zueinander innerhalb dieses Bezugssystems (dem des Abbildungsgerätes) anzugeben. Die Art des gewählten Koordinatensystems ist frei wählbar. Es ist z.B. möglich, kartesische, Zylinder- oder Kugelkoordinaten zu verwenden.

In einem weiteren Verfahrensschritt werden die 2D-Bilddaten basierend auf einer Abbildungstransfomationsfunktion bearbeitet. Diese Abbildungstransformationsfunktion beschreibt eine Abbildungsfunktion des zur Generierung des 2D-Bilddatensatzes verwendeten Abbildungsgerätes, die wiederum einen räumlichen Zusammenhang zwischen der tatsächlichen Lage von abgebildeten Punkten im Raum und ihrem Abbildungsort bei einer Aufnahme beschreibt.

Die Abbildungsfuktion bei Verwendung eines fächerförmigen Abbildungsstrahles unterscheidet sich wesentlich von der Abbildungsfunktion eines kegelförmigen Abbildungsstrahles, wie er z.B. bei der Erstellung fluoroskopischer Bilder verwendet wird. Der wesentliche Unterschied liegt darin, dass bei der Abbildung von Punkten mittels eines fächerförmigen Abbildungsstrahles eine Fächerprojektion vorgenommen wird, wohingegen bei einem kegelförmigen Abbildungsstrahl eine Kegelprojektion erfolgt. Bei einer Aufnahme mit einem fächerförmigen Abbildungsstrahl wird näherungsweise eine fächerförmige Ebene, d.h., eine zweidimensionale Fläche mit X und Y Koordinaten, durchstrahlt, und alle so abgebildeten Punkte werden projiziert, wobei die projizierten Punkte näherungsweise alle auf einer Geraden liegen. Im Gegensatz dazu wird bei einer Aufnahme mit einem kegelförmigen Abbildungsstrahl ein dreidimensionaler Raumabschnitt durchleuchtet, und die in diesem Körper befindlichen Punkte werden auf eine zweidimensionale kreisförmige Fläche abgebildet. Betrachtet man also die erhaltenen Projektionen (Fächerstrahlprojektion und Kcgelstrahlprojektion), so ist ersichtlich, dass die Projektion bei der Fächerprojektion niederdimensionaler ist als die Projektion bei der Kegelprojektion. Die Abbildungstranstformationsfunktion trägt dieser speziellen Geometrie, die bei einer Fächerprojektion auftritt, Rechnung und bevorzugt wird die beschriebene Näherung der Ermittlung der Abbildungstransformationsfunktion zugrunde gelegt. Weitere Parameter, die die fächerförmigen Abbildungsstrahlen weiter charakterisieren, können vorteilhaft der ebene Auffächerungswinkel α (im Gegensatz zu einem Raumwinkel, der die Öffnung eines Kegelstrahles beschreibt) und der Abstand zwischen der Strahlungsquelle und dem Detektor bzw. der linear ausgebildeten Detektionseinheit sein. Dieser Abstand kann z.B. als die minimale Wegstrecke definiert werden, die ein einzelner Strahl von der Strahlungsquelle zum Detektionsort zurücklegt.

Bei dem erfindungsgemäßen Verfahren zum Registrieren eines mit fächerförmigen Abbildungsstrahlen generierten 2D-Bilddatensatzes werden die 2D-Bilddaten basierend auf der beschriebenen Raumtransformationsfunktion und basierend auf der beschriebenen Abbildungstransformationsfunktion bearbeitet. Dies setzt voraus, dass die Raumtransformationsfunktion und die Abbildungstransformationsfunktion bekannt sind. Diese können in Form eines Datensatzes vorliegen, es ist aber auch möglich, dass diese durch eine Parametereingabe festgelegt werden (z.B. durch Eingabe des verwendeten Auffacherungswinkels α und des Abstandes der Strahlungsquelle zum Detektor).

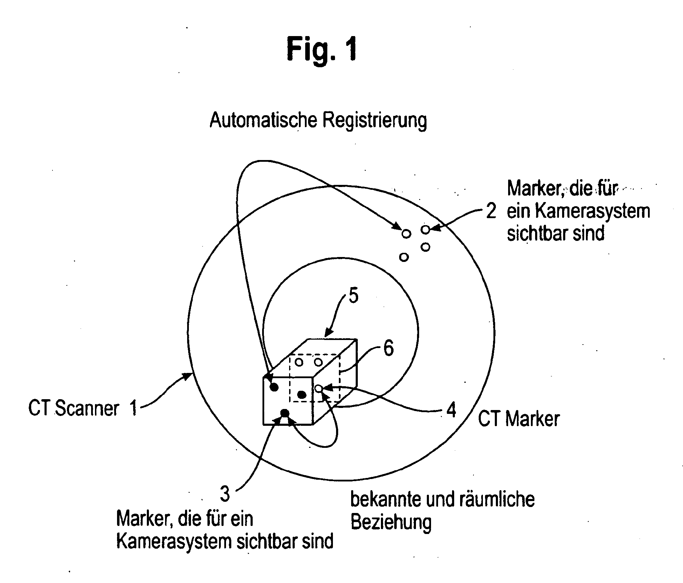

Erfindungsgemäß werden die Abbildungstranslonnationsfunktion und die Raumtransiolmationsfunktion erst ermittelt werden. Dies ist insbesondere dann erforderlich, wenn ein Abbildungsgerät verwendet wird, dessen Geometrie nicht exakt spezifiziert ist. In diesem Fall ist es möglich, eine Referenzmessung mittels eines Referenzkörpers durchzuführen, der es erlaubt, sowohl die Abbildungstransformationsfunktion als auch die Raumtransformationsfunktion zu ermitteln. Zu diesem Zweck ist der Referenzkörper mit einer Mehrzahl von Punkten ausgestattet, deren Position im Raum in Bezug auf das Abbildungsgerät bekannt ist. Zum anderen können diese Punkte bei einer Abbildung mit einem fächerförmigen Abbildungsstrahl in der Abbildungsebene bzw. genauer Abbildungsgerade zugeordnet werden. Eine eingehende Beschreibung des Ermittelns der Abbildungstransiomationsfunktion und der Raumtransformationsfunktionmit Hilfe eines solchen Referenzkörpers wird weiter unten unter Bezugnahme auf Fig. 1 beschrieben.

Gemäß einer bevorzugten Ausführungsform werden zur Ermittlung der Abbildungsfunktion geometrische Projektionen von abzubildenden Punkten auf eine Richtung der Auffächerung des Fächcrstrahls (d.h. auf die Abbildungsgerade) berücksichtigt. Die abzubildenden Punkte befinden sich dabei alle in der Ebene, die von dem fächerförmigen Abbildungsstrahl überstrichen bzw. durchleuchtet wird. Die Höhe der abzubildenden Punkte über der Detektionsgeraden ist dabei bevorzugt unterschiedlich. Bevorzugt werden mindestens drei verschiedene Punkte zur Ermittlung der Abbildungsfunktion und deren Projektionen auf die Abbildungsgerade verwendet. Die Projektion wird dabei so durchgeführt, dass für alle Strahlen des fächerförmigen Abbildungsstrahls deren jeweiliger Strahlengang betrachtet wird, aus dem sich der Abbildungsort von bestrahlten bzw. durchleuchteten Punkten eines Körpers ergibt. Die Gerade, auf die alle Punkte abgebildet werden, definiert die Richtung der Auffächerung des Fächcrstrahls.

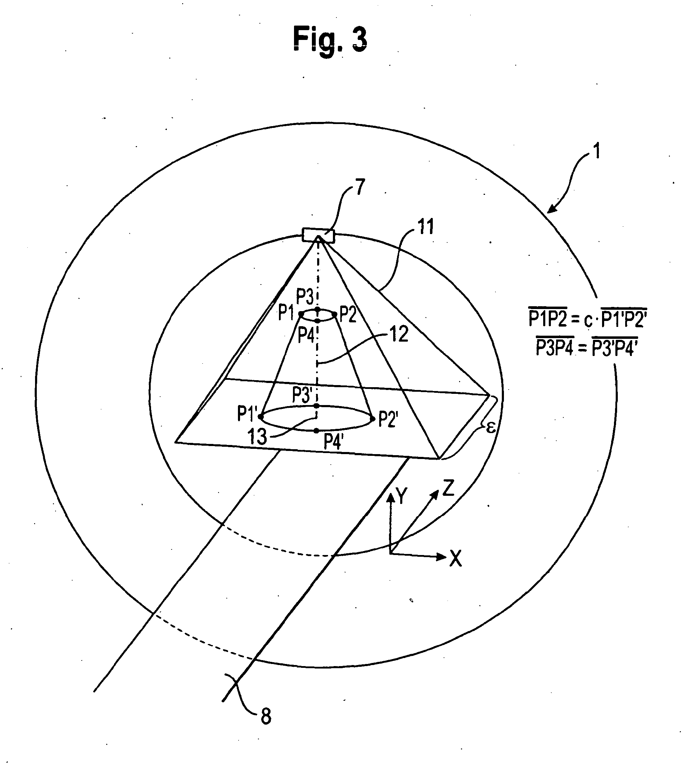

Gemäß einer bevorzugten Ausführungsform umfasst die Ermittlung der Abbildungsfunktion die Ermittlung eines Proportionalitätsfaktors. Dieser beschreibt einen Zusammenhang zwischen räumlichen Relativpositionen von abzubildenden Punkten zueinander in einer bestimmten Höhe über einer Detektionsebene des Abbildungsgerätes und relativen Detektionspositionen der abgebildeten Punkte zueinander in Richtung der Auffächerung. Bei dieser Detektionsebene handelt es sich um einen schmalen Detektionsstreifen (Abbildungsgerade). Die Längsrichtung dieses Detektionsstreifens entspricht der Richtung der Auffächerung des fächerförmigen Abbildungsstrahles. In der Richtung orthogonal zu dieser Auffächerungsrichtung weist der fächerförmige Abbildungsstrahl - wie oben bereits beschreiben - nur eine vernachlässigbare Aufweitung auf. Die Detektionsebene kann deshalb aus einer linearen Anordnung von kleinsten Detektionseinheiten bestehen (z.B. aus einem Zeilendetektor). Dennoch ist es selbstverständlich möglich, auch bei einer schmalen Detektionsebene aufgrund der ebenen Eigenschaften die Höhe eines Punktes über dieser Ebene anzugeben. Befinden sich bei einer Abbildung, die mittels eines fächerförmigen Abbildungsstrahls vorgenommen wird, zwei Punkte in der durchstrahlten Ebene, und befinden sich diese Punkte auf der selben Höhe über der Detektionsebene des Abbildungsgerätes, und sind diese beiden Punkte symmetrisch bezüglich der Strahlachse angeordnet (die Strahlachse entspricht einem Strahl, der bei symmetrischer Gesamtanordnung von der Strahlungsquelle ausgehend die Detektionsebene in kürzestmölichem Abstand erreicht), so ist aufgrund der Symmetrieverhältnisse unmittelbar klar, dass die Abbildungsorte der Punkte in der Detektionscbene sich ebenfalls symmetrisch zum Mittelpunkt befinden. Bei dieser konkreten Form der Abbildung ergibt sich das Vorhandensein eines Proportionalitätsfaktors unmittelbar aus den Strahlensätzen der Mathematik. Die geometrischen Verhältnisse bei einer Abbildung mittels eines fächerförmigen Abbildungsstrahles werden weiter unten unter Bezugnahme auf Fig. 3 eingehender beschrieben.

Die Ermittlung des Proportionalitätsfaktors kann explizit oder implizit erfolgen. In letztgcnanntem Fall wird er nicht explizit ausgegeben, sondern ist implizit durch Werte einer Abbildungsmatrix gegeben.

Das ertindungsgemäße Verfahren zum Registrieren eines mit fächertörmigen Abbildungsstrahlen generierten 2D-Bilddatensatzes im Bereich der Medizin kann nun bei einem Verfahren zum automatischen Registrieren eines Körpers basierend auf 2D-Bilddaten, die zum Einsatz in medizinischen Navigationssystemen geeignet sind, zur Anwendung gelangen. Zu diesem Zweck werden die Verfahrensschritte des Verfahrens zum Registrieren z.B. mit der folgenden Sequenz von Verfahrensschritten gemäß einer Ausführungsform kombiniert: Zunächst wird ein fächerförmiger Abbildungsstrahl von einem Abbildungsgerät erzeugt. Der fächerförmige Abbildungsstrahl hat dieselben Eigenschaften, wie es oben allgemein beschrieben wurde. Insbesondere geht der fächerförmige Abbildungsstrahl von einer idealisierten punktförmigen Strahlungsquelle aus und ist im Wesentlichen nur in einer Richtung, der Auffächerungsrichtung, stark aufgeweitet. In einem nächsten Schritt wird ein Körper durch den fächerförmigen Abbildungsstrahl abgebildet, wobei der fächerförmige Abbildungsstrahl nach Durchtritt durch den Körper entlang seiner Auffächerungsrichtung detektiert wird. Bei diesem Körper kann es sich um einen Gegenstand oder aber um einen Patienten oder bestimmte Körperteile eines Patienten handeln. Anschließend wird aus mehreren zueinander räumlich verschobenen Aufnahmen des Körpers mittels des fächerförmigen Abbildungsstrahls ein 2D-Bilddatensatz zusammengesetzt. Zu diesem Zweck ist es einerseits möglich, die Strahlenquelle über den Körper hinweg zu bewegen, so dass mehreren unterschiedliche Aufnahmen entstehen. Alternativ ist es auch möglich, dass die Strahlungsquelle im Raum fix bleibt, und der Körper oder Gegenstand unter der Strahlungsquelle schrittweise hindurchbewegt wird, wobei in jeder Ruheposition des Körpers eine Aufnahme mittels des fächerförmigen Abbildungsstrahles gemacht wird. Entscheidend ist die Relativbewegung zwischen Abbildungsgerät und Körper. Bei den verschobenen 1 D-Aurnahmen handelt es sich um zueinander parallel verschobene Aufnahmen, die jeweils um einen definierten Betrag in einer Verschieberichtung zueinander versetzt sind. Bevorzugt erfolgt die Verschiebung in einer Richtung orthogonal zur Auffächerungsrichtung des fächerförmigen Abbildungsstrahles. Der auf diese Weise aus 1D-Daten zusammengesetzte gewonnene 2D-Bilddatensatz wird nun dem erfindungsgemäßen Verfahren zum Registrieren eines mit fächerförmigen Abbildungsstrahlen generierten 2D-Bilddatensatzes unterzogen wie oben beschrieben.

Gemäß des Verfahrens zum automatischen Registrieren eines Körpers basierend auf 2D-Bilddaten wird ein Referenzkörper abgebildet. Dieser Referenzkörper wird zum einen zur Ermittlung einer räumlichen Relativposition zwischen dem Reterenzkörper und dem Abbildungsgerät zwecks Bestimmung der Raumtransformationsfunktion verwendet. Zum anderen wird der Referenzkörpcr zur Ermittlung der Abbildungsfunktion des Abbildungsgerätes, die einen räumlichen Zusammenhang zwischen der tatsächlichen Lage von abzubildenden Punkten im Raum und deren Abbildungsort beschreibt, verwendet. Gemäß einer bevorzugten Ausführungsform wird ein Referenzkörper in Quaderform verwendet. Es kann aber auch ein runder Relerenzkörper verwendet werden. Der Referenzkörper weist verschiedene Referenzpunkte auf. Einige dieser Referenzpunkte sind bei der Abbildung mittels des fächerförmigen Abbildungsstrahles identifizierbar d.h. abbildbar. Bei einem quaderförmigen Referenzkörper befinden sich die Referenzpunkte bevorzugt auf verschiedenen Seiten des Quaders oder an Ecken des Quaders und sind bevorzugt aus Materialien mit hohem Z-Wert (Materialien mit hoher Ordnungszahl, wie zum Beispiel Wolfram) hergestellt. Sie können in dem erzeugten Bild eindeutig wiedererkannt und zugeordnet werden. 7.um anderen ist der Referenzkörper mit weiteren Referenzpunkten versehen, die eine Positionsbestimmung des Referenzkörpers im Raum ermöglichen. Dabei kann es sich um herkömmliche Markereinrichtungen handeln, die an dem Referenzkörper angebracht sind. Dabei handelt es sich entweder um aktive Marker, d.h. solche, die Strahlung aussenden, oder um passive Marker, d.h. solche, die Strahlung reflektieren. Bevorzugt handelt es sich bei der Strahlung um Infrarotstrahlung oder um Ultraschall. Bevorzugt verfügt die Markereinrichtung über drei einzelne Marker, die sich in fester relativer Lage zueinander befinden. Die Position der Positions-Marker und der Abbildungsreferenzpunkte ist bekannt, so dass aus der Position der Marker im Raum auf die Position der Abbildungsreferenzpunkte im Raum geschlossen werden kann. Die Ermittlung der Position des Referenzkörpers im Raum basierend auf den Markereinrichtungen erfolgt in an sich bekannter Weise z.B. mittels eines Kamerasystems, das gleichzeitig mit den Markereinrichtungen des Referenzkörpers mindestens eine weitere Markereinrichtung, die in bekannter Relativposition zu dem Abbildungsgerät angebracht ist, erfasst, und die Relativlage der Markersysteme erlaubt es, die Position der Markereinrichtungen an dem Referenzkörper zu bestimmen. Darauf wird weiter unten unter Bezugnahme auf Fig. 1 noch genauer eingegangen werden. Die Referenzpunkte des Referenzkörpers, die bei der Abbildung mit dem fächerförmigen Abbildungsstrahl identifizierbar abgebildet werden, liegen bevorzugt alle in derselben Ebene, die vom fächerförmigen Abbildungsstrahl überstrichen wird. Die Relativpositionen der Abbildungsreferenzpunkte zueinander sind bekannt.

Gemäß einer bevorzugten Ausführungsform sind die Markereinrichtungen und die Abbildungsreferenzpunkte nicht miteinander identisch, gleichwohl ist aber ihre Relativposition zueinander bekannt. Alternativ ist es auch möglich, dass Referenzpunkte gleichzeitig als Markereinrichtungen und als Abbildungsreferenzpunkte dienen. Z.B. können im Inneren eines reflektierenden Markers Materialien (zum Beispiel Wolfram bei Röntgenaufnahmen) eingebracht sein, die eine Identifikation als Abbildungsreferenzpunkt bei einer Aufnahme mit einem fächerförmigen Abbildungsstrahl erlauben.

Gemäß einer bevorzugten Ausführungsform des Verfahrens zum automatischen Registrieren eines Körpers entspricht die Abbildungsauflösung des Abbildungsgerätes in einer Richtung orthogonal zu der Richtung der Auffächerung in etwa einer Positionsverschiebung, die die Abweichung zwischen der tatsächlichen Position eines abzubildenden Punktes in dieser orthogonalen Richtung und der Position seiner Projektion in dieser orthogonalen Richtung beschreibt. Wie oben bereits ausgeführt, ist der fächerförmige Abbildungsstrahl nur in der Auffächerungsrichtung stark aufgeweitet, wohingegen er in der dazu orthogonalen Richtung nur eine sehr geringe Aufweitung aufweist, die im Prinzip vernachlässigbar klein ist. Bei einer exakten Betrachtung der Abbildungsverhältnisse in der Richtung orthogonal zur Auffächerungsrichtung erkennt man, dass ein Punkt, der sich an einer bestimmten Position in der orthogonalen Richtung im Raum befindet, bei dem Abbildungsprozess leicht in der Richtung orthogonal zur Auffächerungsichtung verschoben wird. Diese Verschiebung ist bei der bevorzugten Ausführungsform jedoch derart gering, dass ein Punkt unabhängig von seiner exakten Position in der Richtung orthogonal zur Auffächerungsrichtung dennoch stets von derselben kleinsten physikalischen Detektionseinheit detektiert wird. D.h., die Abbildungsauflösung des Abbildungsgerätes in der Richtung orthogonal zur Auffächerungsrichtung entspricht größenordnungsmäßig der bei der Abbildung auftretenden Positionsverschiebung des Punktes in der Richtung orthogonal zur Auffächerungsrichtung. Anders ausgedrückt ist die geometrische Strahlqualität des fächerförmigen Abbildungsstrahls so gut, dass Abweichungen von der idealen fächerförmigen Strahlform unterhalb der Detektionsgenauigkeit des Abbildungsgerätes liegen.

Gemäß einer bevorzugten Ausführungsform wird zur Ermittlung der räumlichen Relativposition zwischen dem Referenzkörper und dem Abbildungsgerät ein Kamerasystem verwendet. Dabei kann es sich um ein Kamerasystem handeln, das z.B. im Infrarotbereich oder im Ultraschallbereich arbeitet. Das Kamerasystem erkennt eine erste Markereinrichtung in bekannter relativer Lage zu dem Abbildungsgerät gleichzeitig mit einer zweiten Markereinrichtung (wie oben bereits beschrieben) an dem Referenzkörper. Die erste und die zweite Markereinrichtung umfassen jeweils ein gleichartiges Markersystem. Dabei kann es sich bei den Markern um aktive oder passive Marker handeln, d.h., die Marker strahlen einen bestimmte Strahlungsart aus oder aber sie reflektieren eine bestimmte Strahlungsart. Die erste Markereinrichtung ist bevorzugt an dem Abbildungsgerät selbst fest angebracht. Es ist aber auch möglich, dass sich die erste Markereinrichtung in einer bestimmten Entfernung zum Abbildungsgerät befindet, wobei die relative Lage von Abbildungsgerät und erster Markereinrichtung bekannt ist. Die zweite Markereinrichtung kann von außen an dem Referenzkörper angebracht sein, bevorzugt ist es so, dass die zweite Markereinrichtung in den Referenzkörper integriert ist. Des Weiteren ist eine relative Lage der zweiten Markereinrichtung zu spezifischen Abbildungsmarkern des Referenzkörpers, die bei einer Aufnahme mit dem Abbildungsgerät erkannt werden, bekannt. Das bedeutet, der Abstand der zweiten Markereinrichtung zu den spezifischen Abbildungsmarkern des Referenzkörpers ist konstant, und die richtungsmäßige Orientierung der zweiten Markereinrichtung zu den spezifischen Abbildungsmarkern ist ebenfalls bekannt und ändert sich nicht. Dies ermöglicht es, durch Koordinatentransformationen die relativen Positionen zwischen den spezifischen Abbildungsmarkern und dem Abbildungsgerät zu ermitteln. Gemäß einer bevorzugten Ausftihrungsform sind die Marker der zweiten Markereinrichtung nicht identisch mit den spezifischen Abbildungsmarkern des Referenzkörpers, die bei einer Aufnahme mit dem Abbildungsgerät erkannt werden. Es ist jedoch auch möglich, dass die Marker der zweiten Markereinrichtung zumindest teilweise mit den spezifischen Abbildungstnarkern identisch sind. Dies hängt von der physikalischen Natur des Abbildungsverfahrens bzw. der verwendeten Strahlungsart ab.

Gemäß einer bevorzugten Ausführungsform wird der fächerförmige Abbildungsstrahl mit einem Zeilendetektor, insbesondere einem einzelnen Zeilendetektor, detektiert. Bevorzugt wird lediglich ein einzelner Zeilendetektor verwendet, da dies kostengünstiger ist. Auch ist die Strahlqualität für die Verwendung eines einzelnen Zeilendetektors bei vielen Abbildungsgeräten ausreichend.

Bevorzugt wird bei dem Verfahren zum automatischen Registrieren eines Körpers ein fächerförmiger Röntgenstrahl emittiert, der von einem CT-Scanner ausgesendet wird. Solche fächerförmigen Röntgenstrahlen werden von einem CT-Scanner insbesondere dann ausgestrahlt, wenn Topogramme oder Scoutviews im Vorfeld der eigentlichen CT-Aufnahme erstellt werden sollen, um einen Scanbereich festzulegen. Das erfindungsgemäße Verfahren ermöglicht nun eine zusätzliche Verwendung der so gewonnenen 2D CT-Bilddaten für Registrierungs - und anschließende Navigationszwecke.

Gemäß einem weiteren Aspekt bezieht sich die Erfindung auf ein Computerprogrammprodukt mit einem Programmcode zum Ausführen des oben beschriebenen Verfahrens zum Registrieren eines mit fächerförmigen Abbildungsstrahlen generierten 2D-Bilddatensatzes im Bereich der Medizin. Dabei ist es unerheblich, in welcher Programmiersprache der Programmcode verfasst ist oder auf welchem Medium der Programmcode gespeichert ist.

Gemäß einem weiteren Aspekt bezieht sich die Erfindung auf ein System zur automatischen Registrierung eines Körpers basierend auf 2D-Bilddaten zum Einsatz in medizinischen Navigationssystemen. Medizinische Navigationssysteme werden in der Medizin bevorzugt während Operationen eingesetzt, und erlauben es, insbesondere die Position von chirurgischen Instrumenten genau zu erkennen bzw. diese genau zu positionieren, um auf diese Weise ein besseres Behandlungsergebnis zu erzielen. Dabei kann der behandelnde Arzt anhand einer bildlichen Darstellung z.B. auf einem Monitor die aktuelle Position eines Behandlungsgerätes relativ zu einem Behandlungsobjekt, z.B. dem Körper eines Patienten oder einem Knochen, in Echtzeit verfolgen. Die Bilddaten, aufgrund derer der Arzt navigiert, sind registrierte Bilddaten, d.h., es wurde vom System vorab die genaue Position von Bilddaten in Relation zu einer vorher definierten Referenz (z.B. dem Körper des Patienten im Raum) definiert.

Das System zur automatischen Registrierung eines Körpers basierend auf 2D-Bilddaten zum Einsatz in medizinischen Navigationssystemen weist ein Abbildungsgerät und eine Rcchcneinheit auf.

Das Abbiidungsgerät weist eine Erzeugungseinheit zur Erzeugung eines fächerförmigen Abbildungsstrahles, um einen Körper damit abzubilden, auf, und weist des weiteren eine Detektionseinheit zum Detektieren dieses fächerförmigen Abbildungsstrahls entlang seiner Auffächerungsrichtung nach Durchtritt durch den Körper auf. Bevorzugt umfasst die Erzeugungseinheit eine punktförmige Strahlungsquelle. Diese Strahlungsquelle erzeugt bevorzugt einen gerichteten Strahl. Alternativ ist es auch möglich, durch ein entsprechendes Blendensystem einen gerichteten Abbildungsstrahl zu erzeugen. Die Fächerform des Abbildungsstrahles wird bevorzugt durch Blenden geformt. Bei der Detektionseinheit kann es sich um beliebige bekannte Detektionseinheiten handeln. Die Auffächerungsrichtung des fächerförmigen Abbildungsstrahles ist so zu verstehen, wie dies bereits oben bei der Beschreibung des Verfahren zum Registrieren eines mit fächerförmigen Abbildungsstrahlen generierten 2D-Bilddatensatzes im Bereich der Medizin beschrieben worden ist.

Die Recheneinheit des erfindungsgemäßen Systems zur automatischen Registrierung ist eingerichtet, um aus mehreren räumlich verschobenen Abbildungen eines Körpers einen 2D-Bilddatensatz zusammenzusetzen. Dabei wird der abzubildende Körper hinsichtlich des Abhildungsgerätes in eine Relativbewegung versetzt, d.h., der Körper wird unter dem Abbildungsgerät beispielsweise hindurchgefahren, oder das Abbildungsgerät wird über den Körper hinweggefahren, wobei in bestimmten Positionen, die bevorzugt um einen festen Betrag gegeneinander verschoben sind, eine Aufnahme mit dem fächerförmigen Abbildungsstrahl erzeugt wird. Im Prinzip ist es aber auch möglich, mehrere Erzeugungseinheiten und mehrere Detektionseinheiten nacheinander vorzusehen, die jeweils einen fächerförmigen Abbildungsstrahl erzeugen, um einen Körper damit abzubilden, wobei jeder fächerförmige Abbildungsstrahl eine andere Ebene des Körpers durchleuchtet. Aus Gründen der Effizienz ist aber eine Relativbewegung zwischen Körper und Abbildungsgerät vorzuziehen. Bei jeder Aufnahme mittels des fächerförmigen Abbildungsstrahls wird eine 1D-Zeile des 2D-Bilddatensatzes erzeugt. Aus mehreren Zeilen wird wiederum der 2D-Bilddatensatz zusammengesetzt.

Des Weiteren ist die Recheneinheit eingerichtet, um die 2D-Bilddaten zu registrieren, und zwar basierend auf einer Raumtransformationsfunktion, die eine räumliche Relativposition zwischen den abgebildeten Punkten, die mit einem fächerförmigen Abbildungsstrahl abgebildet worden sind, und dem zur Abbildung verwendeten Abbildungsgerät beschreibt, und basierend auf einer Abbildungstransformationsfunktion, die eine Abbildungsfunktion zur Gcnerierung des 2D-Bilddatensatzes verwendete Abbildungsgerätes beschreibt, die einen räumlichen Zusammenhang zwischen der tatsächlichen Lage von abgebildeten Punkten im Raum und deren Abbildungsort bei einer Aufnahme beschreibt. Hinsichtlich der Charakteristika bei der Raumtransformationsfunktion und der Abbildungstransformationsfunktion wird nach oben verwiesen, wo im Rahmen der Beschreibung des Registrierungsverfahrens bereits ausführlich auf die Abbildungstranformationsfunktion und auf die Raumtransformationsfunktion eingegangen worden ist.

Gemäß einer bevorzugten Ausführungsform weist das System eine Kameraeinheit, eine erste Markereinrichtung und einen Referenzkörper auf. Bei der Kameraeinheit kann es sich z.B. um eine infrarotempfindliche Kameraeinheit oder um eine Ultraschalldetektionseinrichtung handeln. Mittels der Kameraeinheit wird die erste Markereinrichtung erkannt, zu diesem Zweck ist die Markereinrichtung mit entsprechenden Markern, die als aktive Signalgeber oder als Reflektoren arbeiten können, verschen. Die erste Markereinrichtung befindet sich dabei in einer bekannten Position zu dem Abbildungsgerät, bevorzugt ist die erste Markereinrichtung fix mit dem Abbildungsgerät verbunden und definiert ein Koordinatensystem des Abbildungsgerätes. Der Referenzkörper der von dem Abbildungsgerät abgebildet wird, verfügt über eine zweite Markereinrichtung, die von der Kameraeinheit gleichzeitig mit der ersten Markereinrichtung erkannt wird. Des Weiteren weist der Referenzkörper spezifische Abbildungsmarker auf, die bei der Aufnahme mit dem Abbildungsgerät erkennbar sind, wobei sich die zweite Markereinrichtung und die spezifischen Abbildungsmarker in bekannter relativer Lage zueinander befinden. Bevorzugt handelt es sich um mindestens drei spezifische Abbildungsmarker. Mindestens drei spezifische Abbildungsmarker befinden sich in einer Ebene, nämlich der Ebene, die von dem fächerförmigen Abbildungsstrahl durchleuchtet wird. Unter Verwendung der beschriebenen Kameraeinheit, der ersten Markereinrichtung und des Referenzkörpers mit seiner zweiten Markereinrichtung und den spezifischen Abbildungsmarkern ist die Recheneinheit in der Lage, die relative Lage zwischen der ersten und der zweiten Markereinrichtung zu ermitteln, darauf basierend die relative Lage zwischen den spezifischen Abbildungsmarkern und der ersten Markereinrichtung zu ermitteln, die Abbildungsfunktion des Abbildungsgerätes basierend auf einer Aufnahme des Referenzkörpers mit seinen spezifischen Abbildungsmarkern zu ermitteln, die einen räumlichen Zusammenhang zwischen der tatsächlichen Lage von abzubildenden Punkten im Raum und deren Abbildungsort bei der Aufnahme mit dem fächerförmigen Abbildungsstrahl beschreibt, und die so gewonnenen Informationen zur Registrieren des 2D-Bilddatensatzes miteinander zu verknüpfen. Auf Basis des registrierten 2D-Bilddatensatzes ist es anschließend möglich, ein Behandlungsgerät wie z.B. ein Skalpell navigationsunterstützt bei einer Operation einzusetzen. Dazu wird das Behandlungsgerät an bekannter Stelle mit einer Markereinrichtung versehen, die ebenfalls mit dem Kamerasystem - gleichzeitig mit der ersten Markereinrichtung - erkannt wird. Mit einer Recheneinheit wird die Position des Behandlungsgerätes aus den Kameradaten bestimmt und bildlich (in dem registrierten Bilddatensatz) dargestellt.

Gemäß einer bevorzugten Ausführungsform weist der Referenzkörper mindestens drei spezifische Abbildungsmarker auf, die während eines Abbildungsvorganges alle in der Ebene des fächerförmigen Abbildungsstrahls angeordnet sind und somit alle abgebildet werden. Bevorzugt ist die Ebene, die durch die drei spezifischen Abbildungsmarker aufgespannt wird, identisch mit der Hauptebene des fächerförmigen Abbildungsstrahls. Kleinere Abweichungen der Ebenen zueinander sind jedoch tolerabel, solange diese Abweichungen so klein sind, dass sie von der verwendeten Detektionseinrichtung nicht aufgelöst werden können.

Bevorzugt sendet das Abbildungsgerät einen fächerförmigen Abbildungsstrahl aus, der in einer Richtung orthogonal zu der Hauptauffächerungsrichtung in etwa so stark aufgeweitet ist, dass die Aufweitung in etwa dem Auflösungsvermögen der Detektionseinheit in dieser Richtung entspricht. Bei der Verwendung eines Zeilendetektors, insbesondere eines einzeiligen Zeilendetektors bedeutet dies, dass die Strahlqualität des fächerförmigen Abbildungsstrahles in geometrischer Hinsicht sehr hoch ist. Die Aufweitung in der Richtung orthogonal zu der Auffächerungsrichtung ist praktisch vernachlässigbar gering. Positionsverschiebungen von Punkten in der Richtung orthogonal zur Hauptauffächerungsrichtung sind so gering, dass sie mittels der Detektionseinrichtung nicht nachgewiesen werden können.

Gemäß einer bevorzugten Ausführungsform handelt es sich bei dem Abbildungsgerät um einen CT-Scanner, der eingerichtet ist, einen fächerförmigen Röntgenstrahl auszusenden, um diesen mit einem Zeilendetektor zu detektieren. Dies ist oftmals bei herkömmlichen CT-Scannern der Fall, wenn dieser einen Scoutview oder ein Topogramm erzeugt, das dazu benutzt wird, einen Scanbereich für die eigentliche CT-3D-Aufnahme festzulegen.

Die vorliegende Erfindung wird noch besser verstanden werden unter Bezugnahme auf die nachfolgenden Figuren:
- Fig. 1: zeigt ein Setup zur automatischen Registrierung unter Verwendung eines quaderförmigen Referenzkörpers;
- Fig. 2: zeigt eine CT-Fächerstrahlprojektion im Vergleich zu einer Kegelstrahlprojektion bei der Aufnahme eines fluoroskopischen Bildes; und
- Fig. 3: zeigt die geometrischen Verhältnisse bei der Abbildung von Punkten mittels eines fächerförmigen Röntgenstrahls.

Bei dem in Fig. 1 gezeigten Setup zur automatischen Registrierung unter Verwendung eines Referenzkörpers sind die geometrischen Beziehungen zwischen dem Referenzkörper 5 und dem CT-Scanner 1 in teilperspektivischer Darstellung dargestellt. Der CT-Scanner 1 ist in gerader Draufsicht auf den CT-Ring dargestellt, wohingegen der Referenzkörper 5 in Form eines Quaders, der Perspektive erkennen lässt, dargestellt ist. Dies dient zur Vereinfachung der Darstellung. Der quaderförmige Referenzkörper 5 weist zwei unterschiedliche Markersysteme auf: Zum einen verfügt der Referenzkörper über drei CT-Marker 4, die bei einer Abbildung mittels des CT-Scanners erkennbar bzw. identifizierbar sind (sog. spezifische Abbildungsmarker). Diese drei CT-Marker 4 befinden sich in einer Ebene 6, die durch die gestrichelte Linie eines Schnittes im Referenzkörper 5 angedeutet ist. Diese Ebene 6 wird bei einer CT-Aufnahme mittels eines fächerförmigen Abbildungsstrahls von dem Fächerstrahl erfasst, so dass alle CT-Marker 4 abgebildet werden.

Des Weiteren weist der Referenzkörper 5 drei Marker 3 einer Markereinrichtung auf, die für ein Kamerasystem sichtbar sind. Im vorliegenden Beispiel handelt es sich um passive Marker, die Strahlung bestimmter Wellenlänge reflektieren (hier infrarot). Die drei Marker 3, die mit dem Kamerasystem (nicht dargestellt) erkennbar sind, werden im Folgenden als Positions-marker bezeichnet, um sie von den spezifischen Abbildungsmarkern 4 zu unterscheiden. Die Positionsmarker 3 sind auf einer Stirnfläche des quaderförmigen Referenzkörpers angebracht und definieren im Ausführungsbeispiel eine Parallelfläche zu der Fläche, die durch die abbildungsspezitischen Marker 4 aufgespannt wird. Der relative Abstand der Positionsmarker 3 zu den abbildungsspezifischen Markern 4 ist bekannt. Im beschriebenen Ausführungsbeispiel ist dazu insbesondere der Abstand der Stirnfläche zu der Schnittfläche 6 bekannt. Auch die relative Lage der Positionsmarker 3 zueinander ist bekannt sowie die relative Lage der abbildungsspezifischen Marker 4 zueinander. Des Weiteren ist auch die relative Lage mindestens eines Positionsmarkers 3 zu mindestens einem abbildungsspezifischen Marker 4 bekannt. Letztendlich stehen ausreichend Parameter zur Verfügung, die es erlauben, die Position der abbildungsspezifischen Marker 4 bezogen auf oder relativ zu den Positionsmarkern 3 und dem durch diese aufgespannten Bezugssystem zu beschreiben.

Im beschriebenen Ausbildungsbeispiel sind am CT-Scanner 1 Marker 2 befestigt, die ebenfalls von einem Kamerasystem erkannt werden können. Im beschriebenen Ausführungsbeispiel handelt es sich wiederum um passive Marker, d.h., Marker, die einen bestimmten Strahlungstyp (hier: Infrarotstrahlung) reflektieren. Auf Basis der Marker 2 ist es möglich, beliebige Punkte in einem Koordinatensystem des CT-Scanners 1 zu beschreiben. Dabei ist es möglich, absolute Positionsdaten von Punkten in diesem Referenzsystem des CT-Scanners 1 anzugeben. Es ist aber auch möglich, relative Lagen von Punkten in diesem Bezugssystem anzugeben und in Datenform abzuspeichern.

Die Marker 2, die an dem CT-Scanner 1 befestigt sind, und die Positionsmarker 3, die an dem Präferenzkörper 5 angebracht sind, sind derart orientiert, dass sie für ein einziges Kamerasystem (nicht dargestellt) gleichzeitig erkennbar sind. Ist nun die Position der Marker 2 an dem CT-Scanner 1 bekannt, und wird im Rahmen des an sich bekannten automatischen Registrierungsverfährens die Position der Positionsmarker 3 an dem Referenzkörper 5 bestimmt, so kann unter Zuhilfenahme der so bestimmten Position auch auf die räumliche Position der spezifischen Ahbildungsmarker 4 im Referensystem des Abbildungsgerätes (d.h., bezüglich der Marker 2 am CT-Scanner 1) rückgeschlossen werden. Die Funktion, die die Position der spezifischen Abbildungsmarker 4 mit dem Bezugsystem des CT-Scanners 1 verknüpft, ist die bereits beschriebene Raumtransformationsfunktion.

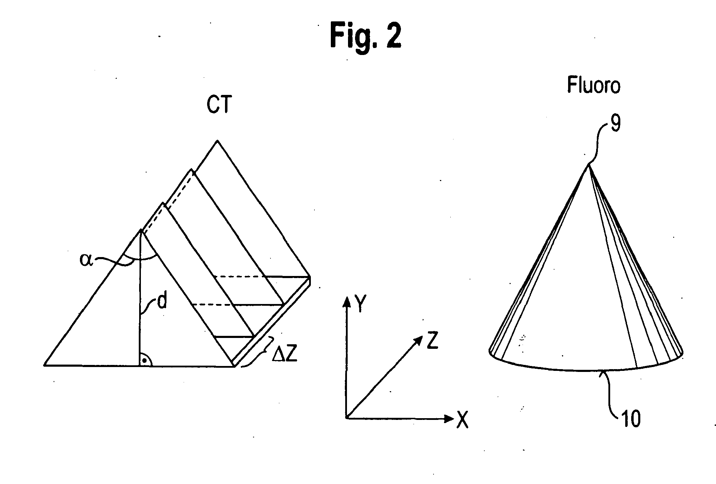

Fig. 2 zeigt die geometrischen Verhältnisse bei einer Fächerstrahlprojektion im Vergleich zu einer Kegelstrahlprojektion. Im vorliegenden Beispiel werden Röntgenstrahlen als Abbildungsstrahlen verwendet und im Vergleich dargestellt. Links in Fig. 2 sind die fächerförmigen Abbildungsstrahlen dargestellt, wie sie bei einem CT-Pre-Scan verwendet werden, es werden die sog. Topogramme oder Scoutviews (2D-Darstcllungen) erzeugt. In der rechten Bildhälfte ist der kegelförmige Abbildungsstrahl einer herkömmlichen Röntgenquelle dargestellt, der ein herkömmliches fluoroskopisches Bild erzeugt.

Die fächerförmigen Abbildungsstrahlen links in Fig. 2 sind idealisiert dargestellt. Idealisiert werden sie auch in dem erfindungsgemäßen Verfahren zur Bildregistrierung behandelt. Der fächerförmige Abbildungsstrahl besitzt eine Aufächerung in der Richtung der Auffächerung X. In dieser Richtung X erfolgt auch die Detektion des fächerförmigen Abbildungsstrahls. Der fächerförmige Abbildungsstrahl besitzt aber nahezu keinerlei Ausdehnung in der orthogonalen Richtung Z, d.h., in der Richtung orthogonal zu der Auffächerungsrichtung X. Ein zweidimensionaler Bilddatensatz wird aus mehreren Einzelbildern in X-Richtung zusammengesetzt. Die einzelnen Bilder sind räumlich parallel zueinander um den Betrag ΔZ verschobcn. Jeder der fächerförmigen Abbildungsstrahlen weist denselben ebenen Auffächerungswinkel α auf. Der Abstand zwischen dem Ausgangspunkt der Strahlen (stimmt praktisch überein mit der Strahlungsquelle) von der Detektionseinheit in Richtung X ist mit d bezeichnet.

In der rechten Bildhälfte von Fig. 2 sind die geometrischen Verhältnisse bei einer fluoroskopischen Aufnahme dargestellt. Ausgehend von der Strahlungsquelle 9 wird ein kegelförmiger Strahl ausgesendet. Der Öffnungswinkel des kegelförmigen Strahles ist ein Raumwinkel. Die Hauptstrahlrichtung verläuft in Richtung der Y-Achse. Es wird ein kegelförmiger Raumabschnitt mit den Röntgenstrahlen durchstrahlt. Die Abbildung ist von sich aus zweidimensionaler Natur, alle Punkte werden in einem kreisförmigen Segment 10 in der XZ-Ebene abgebildet bzw. in diese projiziert.

Der Vergleich der CT-Fächerstrahl-Geometrie mit der kegelstrahltormigen Geometrie der fluoroskopischen Aufnahme zeigt sehr gut, dass die Projektion bei der CT-FächerstrahlAufnahme niederdimensionaler ist. Bei der CT-Fächerstrahlaufnahme erfolgt die Projektion entlang einer Geraden (in Richtung der X-Achse), bei der Projektion mit kegelförmigem Strahlprofil erfolgt die Projektion in eine zweidimensionale Ebene XZ-Ebene.

In Fig. 3 sind die geometrischen Verhältnisse bei der Abbildung bzw. Projektion von einzelnen Punkten dargestellt, wenn ein fächerartiger Abbildungsstrahl verwendet wird. Dargestellt ist wieder der CT-Scanner 1, an dem sich in fester Position eine Röntgenstrahlungsquelle 7 befindet. Diese Röntgenstrahlungsquelle 7 sendet einen fächerförmigen Röntgenstrahl 11 aus. Dabei ist die Aufweitung des Strahles in Richtung orthogonal zu der Auffächerungsrichtung X stark übertrieben dargestellt. Die Aufweitung des Strahles in Richtung Z ist äußerst gering und hier mit ε bezeichnet. Der 2D-Bilddatensatz, der sich aus mehreren zueinander parallel in Richtung Z verschobenen Einzelaufnahmen mit jeweils einem fächerförmigen Abbildungsstrahl zusammensetzt, enthält Einträge in den Richtungen X und Z. Die XZ-Ebene ist im Ausführungsbeispiel die Ebene, in der sich ein relativ zur Röntgenstrahlungsquelle 7 verfahrbarer CT-Tisch 8 befindet.

Betrachtet wird in Fig. 3 nun ein System aus Punkten P1, P2, P3 und P4. Die Punkte P1 und P2 befinden sich in einer bestimmten Höhe über der Detektionsebene (XZ-Ebene), d.h., sie haben dieselbe Y-Koordinate. Legt man den Ursprung des XYZ-Koordinatensystem in den Abbildungspunkt 13, in den ein Strahl entlang der Hauptachse 12 des fächerförmigen Abbildungsstrahls abgebildet wird, so haben die Punkte P1 und P2 dem Betrag nach gleiche X-Koordinaten. Auch sei angenommen, dass P1 und P2 dieselbe Z-Koordinate aufweisen. Die Punkte P1 und P2 werden nun in die Punkte P1' und P2' abgebildet. Diese weisen wiederum identische Y- und Z-Koordinaten auf (im vorliegenden Fall ist Y=Z=0), und die X-Koordinaten von P1' und P2' sind betragsmäßig identisch. Für die Abbildung der Punkte P1 und P2 auf P1' und P2' gilt die folgende Bezichung: *̅P̅*̅1̅*̅P̅*̅2̅*=̅c·**̅P̅*̅1̅'̅*̅P̅*̅2̅'̅. Die Strecke *̅P̅*̅1̅*̅P̅*̅2̅ und die Strecke *̅P̅*̅1̅'̅*̅P̅*̅2̅'̅ sind über den Proportionalitätsfaktor c miteinander verknüpft. Dieselbe Art der Verknüpfung über die Proportionalitätskonstante c gilt für alle Strecken zwischen Punkten Pa und Pb, die dieselbe Y-Koordinate und betragsmäßig identische X-Koordinaten aufweisen. Befinden sich die abzubildenden Punkte Pa und Pb in einer anderen Höhe über der Detektionsebene (XZ-Ebene), d.h., haben sie eine andere Y-Koordinate als im eben beschricbenen Beispiel, so verändert sich der Proportionalitätsfaktor c. Der Proportionalitätsfaktor c ist somit eine Funktion der Höhe (y-Koordinate in Fig. 3) bzw. des Abstandes des abzubildenden Punktes zur Strahlenquelle.

Gemäß einer bevorzugten Ausführungsform wird der Proportionalitätsfaktor c nicht explizit bestimmt, es wird stattdessen das Abbildungsgesetz ermittelt. Dazu wird eine Abbildungsmatrix bestimmt. Für die verwendete Fächerstrahlabbildung hat eine solche Abbildungsmatrix eine einfachere Form als zum Beispiel für Fluoro Bilder. Die Abbildungsmatrix für eine Fächerstrahlabbildung ist insbesondere niederdimensionaler.

Nachdem die geometrischen Verhältnisse bei einer Projektion auf die X-Richtung beschrieben worden sind, soll nun kurz auf die geometrischen Verhältnisse in der weit stärker fokussierten bzw. kaum aufgeweiteten Strahlrichtung Z eingegangen werden. Betrachtet werden die Punkte P3 und P4, die dieselbe X- und Y-Koordinate aufweisen, und die abgebildet werden sollen. Die Punkte P3 und P4 unterscheiden sich allerdings leicht in ihrer Z-Koordinate. Bei einer Abbildung der Punkte P3 und P4 auf die Punkte P3' und P4' in der XZ-Ebene ist zu erkennen, dass die X- und Y-Position der Punkte P3' und P4' wiederum identisch ist, dass sich die Z-Koordinate allerdings leicht unterscheidet. Dieser Unterschied in der Z-Koordinate der Punkte P3' und P4' ist allerdings extrem klein und liegt unterhalb des Auflösungsvermögens des Detektors, der in der beispielhaft beschriebenen Ausführungsiform verwendet wird. Der maximale Abstand von abgebildeten Punkten P3' und P4' in Z-Richtung ist ε. Das Auflösunggsvermögen des verwendeten Zeilendetektors ist jedoch größer oder gleich s, d.h., die Punkte P3' und P4' werden bei der Detektion auf alle Fälle demselben Z-Wert zugeordnet. Näherungsweise gilt somit folgende Beziehung: Strecke *̅P̅*̅3̅*̅P̅*̅4̅ = *̅P̅*̅3̅'*̅P̅*̅4̅'̅.

Obwohl in Fig. 3 die Abbildung der Punkte P1, P2, P3 und P4 auf die Punkte P1', P2', P3' und P4' beschrieben wurde, ist klar, dass auch bei einer Projektion von drei Punkten, wie sie z.B. die spezifischen Abbildungsmarker 4 des Referenzkörpers 5 darstellen, die geometrischen Verhältnisse im Prinzip dieselben sind. Aus den geometrischen Verhältnissen bei der Abbildung der spezifischen Abbildungsmarker 4 mittels eines fächerförmigen Abbildungsstrahles 11 kann somit auf die Abbildungstransformationsfunktion geschlossen werden.

Zur vereinfachenden Beschreibung der geometrischen Strahlenverhältnisse wurde der Ursprung des Koordinatensystems XYZ in die Detektionsebene XZ gelegt. Alternativ ist es möglich, dass die Position der Röntgenstrahlungsquelle 7 als Koordinatenursprung gewählt wird. Es ist problemlos möglich, die Positionen der abzubildenden Punkte und die Orte ihrer Projektion von einem Koordinatensystem in ein anderes Koordinatensystem umzuwandeln.

## Patentansprüche

1. Verfahren zum Registrieren eines mit fächerförmigen Abbildungsstrahlen (11) generierten, zueinander räumlich verschobene Aufnahmen umfassenden 2D-Bilddatensatzes mit einem Körper im Bereich der Medizin, das die folgenden Schritte aufweist:
• Ermitteln, mittels eines Computers, einer Abbildungstransformationsfunktion, die eine Abbildungsfunktion des zur Generierung des 2D-Bilddatensatzes verwendeten Abbildungsgerätes (1) beschreibt, die einen räumlichen Zusammenhang zwischen der tatsächlichen Lage von abgebildeten Punkten (P) in der Ebene des fächerförmigen Abbildungsstrahls und deren Abbildungsort (P') bei jeweils einer der mit fächerförmigen Abbildungsstrahlen generierten Aufnahmen beschreibt,
wobei die Abbildungstransformationsfunktion basierend auf einer Aufnahme eines Referenzkörpers (5) mit spezifischen Abbildungsmarkern (4) ermittelt wird,
wobei der Referenzkörper (5) mindestens drei spezifische Abbildungsmarker (4) aufweist, die während eines Abbildungsvorganges alle in der Ebene (Y) des fächerförmigen Abbildungsstrahls (11) angeordnet sind und somit alle abgebildet werden, und wobei der Referenzkörper (5) und das Abbildungsgerät (1) Positionsmarker (2, 3) aufweisen, wobei wenigstens einer der Positionsmarker des Referenzkörpers eine bekannte Position relativ zu wenigstens einem Abbildungsmarker (4) hat, wobei eine Raumtransformationsfunktion, die eine räumliche Relativposition zwischen Punkten (P), die mit einem fächerförmigen Abbildungsstrahl (11) auf einer Aufnahme abgebildet worden sind, und dem zur Abbildung verwendeten Abbildungsgerät (1) beschreibt, basierend auf der Bestimmung der Position der Positionsmarker (3) des Referenzkörpers (5) relativ zur Position der Positionsmarker (2) des Abbildungsgeräts (1) ermittelt wird; und
• Bearbeiten der 2D-Bilddaten mittels eines Computers basierend auf der so ermittelten Abbildungstransformationsfunktion und der so ermittelten Raumtransformationsfunktion.

2. Verfahren gemäß Anspruch 1, wobei zur Ermittlung der Abbildungsfunktion geometrische Projektionen von abzubildenden Punkten (P) auf eine Richtung der Auffächerung (X) des Fächerstrahls berücksichtigt werden.

3. Verfahren gemäß Anspruch 2, wobei die Ermittlung der Abbildungsfunktion die Ermittlung eines Proportionalitätsfaktors (c) umfasst, der einen Zusammenhang zwischen räumlichen Relativpositionen von abzubildenden Punkten (P) zueinander in einer bestimmten Höhe über einer Detektionsebene des Abbildungsgerätes (1) und relativen Detektionspositionen der abgebildeten Punkte (P') zueinander in Richtung der Auffächerung(X) beschreibt.

4. Verfahren zum automatischen Registrieren eines Körpers basierend auf 2D-Bilddaten, die zum Einsatz in medizinischen Navigationssystemen geeignet sind, das die Verfahrensschritte des Verfahrens gemäß einem der Ansprüche 1 bis 3 und die folgenden Schritte aufweist:
• ein fächerförmiger Abbildungsstrahl (11) wird von einem Abbildungsgerät (1) erzeugt,
• ein Körper wird durch den fächerförmigen Abbildungsstrahl (11) abgebildet, wobei der fächerförmige Abbildungsstrahl (11) nach Durchtritt durch den Körper entlang seiner Auffächerungsrichtung (X) detektiert wird, und
• aus mehreren zueinander räumlich verschobenen Aufnahmen des Körpers mittels des fächerförmigen Abbildungsstrahls (11) wird ein 2D-Bilddatensatz zusammengesetzt,
sodass basierend auf dem 2D-Bilddatensatz navigiert werden kann. wobei ein Referenzkörper (5)
• zur Ermittlung einer räumlichen Relativposition zwischen dem Referenzkörper (5) und dem Abbildungsgerät (1) zwecks Bestimmung der Raumtransformationsfunktion und
• zur Ermittlung der Abbildungsfunktion des Abbildungsgerätes (1), die einen räumlichen Zusammenhang zwischen der tatsächlichen Lage von abzubildenden Punkten (P) im Raum und deren Abbildungsort (P') beschreibt,
abgebildet wird.

5. Verfahren gemäß Anspruch 4, bei dem die Abbildungsauflösung(ε) des Abbildungsgerätes (1) in einer Richtung (Z) orthogonal zu der Richtung der Auffächerung (X) in etwa einer Positionsverschiebung entspricht, die die Abweichung zwischen der tatsächlichen Position eines abzubildenden Punktes in dieser orthogonalen Richtung und der Position seiner Projektion in dieser orthogonalen Richtung beschreibt.

6. Verfahren gemäß einem der Ansprüche 4 bis 5,
• wobei zur Ermittlung der räumlichen Relativposition zwischen dem Referenzkörper (5) und dem Abbildungsgerät(1) ein Kamerasystem verwendet wird,
• wobei das Kamerasystem die Positionsmarker des Abbildungsgeräts (2) in bekannter relativer Lage zu dem Abbildungsgerät (1) gleichzeitig mit zweiten Positionsmarkern (3) an dem Referenzkörper (5) erkennt, und
• wobei eine relative Lage der zweiten Markereinrichtung (3) zu spezifischen Abbildungsmarkern (4) des Referenzkörpers (5), die bei einer Aufnahme mit dem Abbildungsgerät (1) erkannt werden, bekannt ist, und
• wobei durch Koordinatentransformationen die relativen Positionen zwischen den spezifischen Abbildungsmarkern (4) und dem Abbildungsgerät (1) ermittelt werden.

7. Verfahren gemäß einem der Ansprüche 4 bis 6, wobei der fächerförmige Abbildungsstrahl (11) mit einem Zeilendetektor, insbesondere einem einzelnen Zeilendetektor, detektiert wird.

8. Verfahren gemäß einem der Ansprüche 4 bis 7, wobei ein fächerförmiger Röntgenstrahl (11) emittiert wird, der von einem CT-Scanner (1) emittiert wird.

9. Computerprogrammprodukt mit einem Programmcode zum Ausführen des Verfahrens gemäß einem der Ansprüche 1 bis 4.

10. System zur automatischen Registrierung eines Körpers basierend auf einem mit fächerformigen Abbildungstrahlen generierten, zueinander räumlich verschobene Aufnahmen umfassenden 2D-Bilddatensalzes mit dem Körper zum Einsatz in medizinischen Navigationssystemen, das folgendes aufweist:
• ein Abbildungsgerät (1)
o mit einer Erzeugungseinheit (7) zur Erzeugung eines fächerförmigen Abbildungsstrahles (11), um einen Körper damit abzubilden, wobei die Abbildungsstrahlen (11) von einem CT-Scanner (1) emittierte Röntgenstrahlen sind, und
o mit einer Detektionseinheit zum Detektieren dieses fächerförmigen Abbildungsstrahls entlang seiner Auffächerungsrichtung nach Durchtritt durch den Körper, und
• eine Recheneinheit, die eingerichtet ist,
o eine Abbildungstransformationsfunktion, die eine Abbildungsfunktion des zur Generierung eines 2D-Bilddatensatzes verwendeten Abbildungsgerätes (1) beschreibt, wobei der 2D-Bilddatensatz aus mehreren räumlich verschobenen Aufnahmen eines Körpers zusammengesetzt wird, wobei die Abbildungsfunktion einen räumlichen Zusammenhang zwischen der tatsächlichen Lage von abgebildeten Punkten (P) in der Ebene des fächerförmigen und deren Abbildungsort (P') bei jeweils einer der mit fächerförmigen Abbildungstrahlen generierten Aufhahmen beschreibt, basierend auf einer Aufnahme eines Referenzkörpers (5) mit spezifischen Abbildungsmarkem (4) zu ermitteln, wobei der Referenzkörper (5) mindestens drei spezifische Abbildungsmarker (4) aufweist, die während eines Abbildungsvorganges alle in der Ebene (Y) eines fächerförmigen Abbildungsstrahls (11) angeordnet sind und somit alle abgebildet werden, und wobei der Referenzkörper (5) und das Abbildungsgerät (1) Positionsmarker (2, 3) aufweisen, wobei wenigstens einer der Positionsmarker des Referenzkörpers eine bekannte Position relativ zu wenigstens einem Abbildungsmarker (4) hat, und
o eine Raumtransformationsfunktion, die eine räumliche Relativposition zwischen Punkten (P), die mit einem fächerförmigen Abbildungsstrahl (11) auf einer Aufnahme abgebildet worden sind, und dem zur Abbildung verwendeten Abbildungsgerät (1) beschreibt, basierend auf der Bestimmung der Position der Positionsmarker (3) des Referenzkörpers (5) relativ zur Position der Positionsmarker (2) des Abbildungsgeräts (1) zu ermitteln und die 2D-Bilddaten basierend auf der so ermittelten Abbildungstransformationsfunktion und der so ermittelnden Raumtransformationsfunktion zu bearbeiten.

11. System gemäß Anspruch 10, das folgendes aufweist:
• eine Kameraeinheit;
• erste Positionsmarker (2), die sich in bekannter Position zu dem Abbildungsgerät (1) befindet und die von der Kameraeinheit erkannt wird;
• einen Referenzkörper (5), der von dem Abbildungsgerät (1) abgebildet wird,
o der zweite Positionsmarker (3) aufweist, die von der Kameraeinheit gleichzeitig mit der ersten Markereinrichtung (2) erkannt werden, und
o der spezifische Abbildungsmarker (4) aufweist, die bei einer Aufnahme mit dem Abbildungsgerät (1) erkennbar sind,
wobei sich die zweiten Positionsmarker (3) und die spezifischen Abbildungsmarker (4) in bekannter relativer Lage zueinander befinden;
wobei die Recheneinheit,
o die relative Lage zwischen den ersten (2) und den zweiten (3) Positionsmarkern tung (2, 3) ermittelt,
o darauf basierend die relative Lage zwischen den spezifischen Abbildungs-markern (4) und den ersten Positionsmarkern (2) ermittelt, und
o die so gewonnenen Informationen zum Registrieren des 2D-Bilddatensatzes miteinander verknüpft.

12. System gemäß einem der Ansprüche 10 bis 11, wobei das Abbildungsgerät (1) einen fächerförmigen Abbildungsstrahl (11) aussendet, der in einer Richtung (Z) orthogonal zu der Hauptauffächerungsrichtung(X) in etwa so stark aufgeweitet ist, dass die Aufweitung dem Auflösungsvermögen (ε) der Detektionseinheit in dieser Richtung entspricht und/oder wobei es sich bei dem Abbildungsgerät um einen CT-Scanner (1) handelt, der eingerichtet ist, einen fächerförmigen Röntgenstrahl (11) auszusenden und diesen mit einem Zeilendetektor zu detektieren.

## Claims

1. A method for registering a two-dimensional image data set comprising a body, generated using fan-shaped imaging rays (11) and comprising recordings which are spatially shifted relative to each other, in the medical field, comprising the following steps:
• ascertaining, by means of a computer, an imaging transformation function which describes an imaging function of the imaging apparatus (1) used for generating the two-dimensional image data set, which describes a spatial relationship between the actual position of imaged points (P) in the plane of the fan-shaped imaging ray and their imaging location (P') each in one of the recordings generated using fan-shaped imaging rays,
• wherein the imaging transformation function is ascertained on the basis of a recording of a reference body (5) comprising specific imaging markers (4),
• wherein the reference body (5) comprises at least three specific imaging markers (4) which are all arranged in the plane (Y) of the fan-shaped imaging ray (11) during an imaging process and are thus all imaged, and wherein the reference body (5) and the imaging apparatus (1) comprise position markers (2, 3), wherein at least one of the position markers of the reference body has a known position relative to at least one imaging marker (4), wherein a spatial transformation function which describes a spatial relative position between points (P) which have been imaged on a recording using a fan-shaped imaging ray (11) and the imaging apparatus (1) used for imaging is ascertained on the basis of the determined position of the position markers (3) of the reference body (5) relative to the position of the position markers (2) of the imaging apparatus (1); and
• processing the two-dimensional image data, by means of a computer, on the basis of the imaging transformation function and spatial transformation function ascertained in this way.

2. The method in accordance with claim 1, wherein geometric projections of points (P) to be imaged, onto a fanning direction (X) of the fanned ray, are taken into account for ascertaining the imaging function.

3. The method in accordance with claim 2, wherein ascertaining the imaging function comprises ascertaining a proportionality factor (c) which describes a relationship between spatial positions of points (P) to be imaged, relative to each other, at a certain height above a detection plane of the imaging apparatus (1), and detection positions of the imaged points (P'), relative to each other, in the fanning direction (X).

4. A method for automatically registering a body on the basis of two-dimensional image data which are suitable for use in medical navigation systems, comprising the method steps of the method in accordance with any one of claims 1 to 3 and the following steps:
• a fan-shaped imaging ray (11) is generated by an imaging apparatus (1);
• a body is imaged by the fan-shaped imaging ray (11), wherein once it has passed through the body, the fan-shaped imaging ray (11) is detected along its fanning direction (X); and
• a two-dimensional image data set is assembled from a number of recordings of the body by means of the fan-shaped imaging ray (11) which are spatially shifted relative to each other,
such that it is possible to navigate on the basis of the two-dimensional image data set,
wherein a reference body (5) is imaged
• for ascertaining a spatial relative position between the reference body (5) and the imaging apparatus (1) for the purpose of determining the spatial transformation function and
• for ascertaining the imaging function of the imaging apparatus (1), which describes a spatial relationship between the actual spatial position of points (P) to be imaged and their imaging location (P').

5. The method in accordance with claim 4, wherein the imaging resolution (ε) of the imaging apparatus (1) in a direction (Z) orthogonal to the fanning direction (X) approximately corresponds to a shift in position which describes the deviation between the actual position of a point to be imaged in said orthogonal direction and the position of its projection in said orthogonal direction.

6. The method in accordance with any one of claims 4 to 5, wherein:
• a camera system is used for ascertaining the spatial relative position between the reference body (5) and the imaging apparatus (1);
• the camera system identifies the position markers (2) of the imaging apparatus in a known position relative to the imaging apparatus (1), at the same time as second position markers (3) on the reference body (5); and
• a position of the second marker device (3) relative to specific imaging markers (4) of the reference body (5), which are identified by the imaging apparatus (1) during imaging, is known; and
• the relative positions between the specific imaging markers (4) and the imaging apparatus (1) are ascertained by co-ordinate transformations.

7. The method in accordance with any one of claims 4 to 6, wherein the fan-shaped imaging ray (11) is detected using a line detector, in particular an individual line detector.

8. The method in accordance with any one of claims 4 to 7, wherein a fan-shaped x-ray (11) is emitted from a CT scanner (1).

9. A computer program product comprising a program code for performing the method in accordance with any one of claims I to 4.

10. A system for automatically registering a body on the basis of two-dimensional image data, generated using fan-shaped imaging rays and comprising recordings which are spatially shifted relative to each other, for use in medical navigation systems, comprising:
• an imaging apparatus (1), comprising:
○ a generating unit (7) for generating a fan-shaped imaging ray (11) in order to image a body using the same, wherein the imaging rays (11) are x-rays emitted from a CT scanner (1); and
○ a detection unit for detecting said fan-shaped imaging ray along its fanning direction, once it has passed through the body; and
• a computational unit, which is configured to:
o ascertain, on the basis of a recording of a reference body (5) comprising specific imaging markers (4), an imaging transformation function which describes an imaging function of the imaging apparatus (1) used for generating a two-dimensional image data set, wherein the two-dimensional image data set is assembled from a number of spatially shifted recordings of a body, wherein the imaging function describes a spatial relationship between the actual position of imaged points (P) in the plane of the fan-shaped imaging ray and their imaging location (P') each in one of the recordings generated using fan-shaped imaging rays, wherein the reference body (5) comprises at least three specific imaging markers (4) which are all arranged in the plane (Y) of a fan-shaped imaging ray (11) during an imaging process and are thus all imaged, and wherein the reference body (5) and the imaging apparatus (1) comprise position markers (2, 3), wherein at least one of the position markers of the reference body has a known position relative to at least one imaging marker (4); and
○ ascertain, on the basis of the determined position of the position markers (3) of the reference body (5) relative to the position of the position markers (2) of the imaging apparatus (1), a spatial transformation function which describes a spatial relative position between points (P) which have been imaged on a recording using a fan-shaped imaging ray (11) and the imaging apparatus (1) used for imaging, and process the two-dimensional image data on the basis of the imaging transformation function and spatial transformation function ascertained in this way.

11. The system in accordance with claim 10, comprising:
• a camera unit;
• first position markers (2) which are situated in a known position relative to the imaging apparatus (1) and are identified by the camera unit;
• a reference body (5) which is imaged by the imaging apparatus (1) and comprises:
○ second position markers (3) which are identified by the camera unit at the same time as the first marker device (2); and
○ specific imaging markers (4) which can be identified by the imaging apparatus (1) during imaging;
wherein the second position markers (3) and the specific imaging markers (4) are situated in a known position relative to each other;
wherein the computational unit:
○ ascertains the relative position between the first (2) and second (3) position markers;
○ ascertains, on the basis of this, the relative position between the specific imaging markers (4) and the first position markers (2); and
○ links together the information acquired in this way for registering the two-dimensional image data set.

12. The system in accordance with any one of claims 10 to 11, wherein the imaging apparatus (1) emits a fan-shaped imaging ray (11) which is flared in a direction (Z) orthogonal to the main fanning direction (X) approximately to such an extent that the flaring corresponds to the resolution (ε) of the detection unit in this direction, and/or wherein the imaging apparatus is a CT scanner (1) which is configured to emit a fan-shaped x-ray (11) and to detect it using a line detector.

## Revendications

1. Procédé pour enregistrer un ensemble de données d'image 2D avec un corps dans le domaine médical, lequel ensemble étant généré avec des rayons d'imagerie en forme d'éventail (11) et incluant des enregistrements spatialement décalés les uns des autres, comportant les étapes suivantes consistant à :
• déterminer, au moyen d'un ordinateur, une fonction de transformation d'imagerie décrivant une fonction d'imagerie de l'appareil d'imagerie (1) utilisé pour générer l'ensemble de données d'image 2D, laquelle fonction d'imagerie décrit une relation spatiale entre la position effective de points (P) reproduits en image dans le plan du rayon d'imagerie en forme d'éventail et leur emplacement d'imagerie (P') sur chaque enregistrement généré avec des rayons d'imagerie en forme d'éventail,
dans lequel la fonction de transformation d'imagerie est déterminée sur la base d'un enregistrement d'un corps de référence (5) avec des marqueurs d'imagerie spécifiques (4),
dans lequel le corps de référence (5) comporte au moins trois marqueurs d'imagerie spécifiques (4) qui sont tous disposés dans le plan (Y) du rayon d'imagerie en forme d'éventail (11) pendant un processus d'imagerie et sont ainsi tous reproduits en image, et dans lequel le corps de référence (5) et l'appareil d'imagerie (1) comportent des marqueurs de position (2, 3), dans lequel au moins un des marqueurs de position du corps de référence a une position connue par rapport à au moins un marqueur d'imagerie (4), dans lequel une fonction de transformation spatiale décrivant une position spatiale relative entre des points (P) qui ont été reproduits en image sur un enregistrement avec un rayon d'imagerie en forme d'éventail (11), et l'appareil d'imagerie (1) utilisé pour l'imagerie, est déterminée sur la base de la détermination de la position des marqueurs de position (3) du corps de référence (5) par rapport à la position des marqueurs de position (2) de l'appareil d'imagerie (1), et
• traiter les données d'image 2D au moyen d'un ordinateur sur la base de la fonction de transformation d'imagerie ainsi déterminée et de la fonction de transformation spatiale ainsi déterminée.

2. Procédé selon la revendication 1, dans lequel des projections géométriques de points (P) à reproduire en image sur une direction de mise en éventail (X) du rayon en éventail sont prises en compte pour déterminer la fonction d'imagerie.

3. Procédé selon la revendication 2, dans lequel la détermination de la fonction d'imagerie inclut la détermination d'un facteur de proportionnalité (c) qui décrit une relation entre des positions spatiales relatives de points (P) à reproduire en image les uns par rapport aux autres à une certaine hauteur au-dessus d'un plan de détection de l'appareil d'imagerie (1) et de positions de détection relatives des points (P') représentés en image les uns par rapport aux autres dans la direction de la mise en éventail (X).

4. Procédé pour enregistrer automatiquement un corps sur la base de données d'image 2D qui sont adaptées pour être utilisées dans des systèmes de navigation médicaux, comportant les étapes de procédé du procédé selon l'une quelconque des revendications 1 à 3 ainsi que les étapes suivantes consistant à :
• générer un rayon d'imagerie en forme d'éventail (11) à partir d'un appareil d'imagerie (1),
• reproduire en image un corps par l'intermédiaire du rayon d'imagerie en forme d'éventail (11), le rayon d'imagerie en forme d'éventail (11) étant détecté après être passé à travers le corps le long de sa direction de mise en éventail (X), et
• assembler un ensemble de données d'image 2D à partir de plusieurs enregistrements du corps spatialement décalés les uns par rapport aux autres au moyen du rayon d'imagerie en forme d'éventail (11),
de telle sorte qu'il est possible de naviguer sur la base de l'ensemble de données d'image 2D, un corps de référence (5) étant reproduit en image
• pour déterminer une position spatiale relative entre le corps de référence (5) et l'appareil d'imagerie (1) afin de déterminer la fonction de transformation spatiale et
• pour déterminer la fonction d'imagerie de l'appareil d'imagerie (1) qui décrit une relation spatiale entre la position effective de points (P) à représenter dans l'espace et leur emplacement d'imagerie (P').

5. Procédé selon la revendication 4, dans lequel la résolution d'imagerie (ε) de l'appareil d'imagerie (1) dans une direction (Z) orthogonale à la direction de la mise en éventail (X) correspond approximativement à un décalage de position qui décrit l'écart entre la position effective du point à représenter en image dans cette direction orthogonale et la position de sa projection dans cette direction orthogonale.

6. Procédé selon l'une quelconque des revendications 4 à 5,
• dans lequel un système de caméra est utilisé pour déterminer la position spatiale relative entre le corps de référence (5) et l'appareil d'imagerie (1),
• dans lequel le système de caméra détecte les marqueurs de position (2) de l'appareil d'imagerie dans une position relative connue par rapport à l'appareil d'imagerie (1) simultanément avec des seconds marqueurs de position (3) sur le corps de référence (5), et
• dans lequel une position relative du second marqueur de position (3) est connue par rapport à des marqueurs d'imagerie spécifiques (4) du corps de référence (5) qui sont détectés lors d'un enregistrement avec l'appareil d'imagerie (1), et
• dans lequel les positions relatives entre les marqueurs d'imagerie spécifiques (4) et l'appareil d'imagerie (1) sont déterminées par des transformations de coordonnées.

7. Procédé selon l'une quelconque des revendications 4 à 6, dans lequel le rayon d'imagerie en forme d'éventail (11) est détecté avec un détecteur linéaire, en particulier un détecteur linéaire unique.

8. Procédé selon l'une quelconque des revendications 4 à 7, dans lequel un rayon X en forme d'éventail (11) est émis à partir d'un tomodensitomètre (1).

9. Produit de programme informatique comportant un code de programme pour la mise en oeuvre du procédé selon l'une quelconque des revendications 1 à 4.

10. Système pour enregistrer automatiquement un corps sur la base d'un ensemble de données d'image 2D avec le corps, lequel ensemble étant généré avec des rayons d'imagerie en forme d'éventail et incluant des enregistrements spatialement décalés les uns des autres, pour être utilisé dans des systèmes de navigation médicaux, comportant les éléments suivants :
• un appareil d'imagerie (1) comportant :
° une unité de génération (7) pour générer un rayon d'imagerie en forme d'éventail (11) afin de reproduire ainsi un corps en image, dans lequel les rayons d'imagerie (11) sont des rayons X émis par un tomodensitomètre (1), et
° une unité de détection pour détecter ce rayon d'imagerie en forme d'éventail le long de sa direction de mise en éventail après être passé à travers le corps, et
• une unité de calcul configurée pour :
° déterminer une fonction de transformation d'imagerie décrivant une fonction d'imagerie de l'appareil d'imagerie (1) utilisé pour générer l'ensemble de données d'image 2D, dans lequel l'ensemble de données d'image 2D est assemblé à partir de plusieurs enregistrements du corps spatialement décalés, dans lequel la fonction d'imagerie décrit une relation spatiale entre la position effective de points (P) à reproduire en image dans le plan du rayon d'imagerie en forme d'éventail et leur emplacement d'imagerie (P') sur chaque enregistrement généré avec des rayons d'imagerie en forme d'éventail, sur la base d'un enregistrement d'un corps de référence (5) avec des marqueurs d'imagerie spécifiques (4), dans lequel le corps de référence (5) comporte au moins trois marqueurs d'imagerie spécifiques (4) qui sont tous disposés dans le plan (Y) d'un rayon d'imagerie en forme d'éventail (11) pendant un processus d'imagerie et qui sont ainsi tous reproduits en image, et dans lequel le corps de référence (5) et l'appareil d'imagerie (1) comportent des marqueurs de position (2, 3), dans lequel au moins un des marqueurs de position du corps de référence a une position connue par rapport à au moins un marqueur d'imagerie (4), et
° déterminer une fonction de transformation spatiale décrivant une position spatiale relative entre des points (P) qui ont été reproduits en image avec un rayon d'imagerie en forme d'éventail (11) sur un enregistrement, et l'appareil d'imagerie (1) utilisé pour l'imagerie, sur la base de la détermination de la position des marqueurs de position (3) du corps de référence (5) par rapport à la position des marqueurs de position (2) de l'appareil d'imagerie (1), et traiter les données d'image 2D sur la base de la fonction de transformation d'imagerie ainsi déterminée et de la fonction de transformation spatiale ainsi déterminée.

11. Système selon la revendication 10, comportant les éléments suivants :
• une unité de caméra,
• un premier marqueur de position (2) situé dans une position connue par rapport à l'appareil d'imagerie (1) et détecté par l'unité de caméra,
• un corps de référence (5) reproduit en image par l'appareil d'imagerie (1), comportant :
° le second marqueur de position (3) qui a été détecté par l'unité de caméra simultanément avec le premier marqueur de position (2), et
° les marqueurs d'imagerie spécifiques (4) qui peuvent être détectés lors d'un enregistrement avec l'appareil d'imagerie (1),
dans lequel les seconds marqueurs de position (3) et les marqueurs d'imagerie spécifiques (4) sont dans des positions relatives connues,
dans lequel l'unité de calcul :
° détermine la position relative entre les premiers (2) et les seconds (3) marqueurs de position,
° détermine, sur la base de cette information, la position relative entre les marqueurs d'imagerie spécifiques (4) et les premiers marqueurs de position (2), et
° assemble les informations ainsi acquises pour l'enregistrement de l'ensemble de données d'image 2D.

12. Système selon l'une quelconque des revendications 10 à 11, dans lequel l'appareil d'imagerie (1) émet un rayon d'imagerie en forme d'éventail (11) qui est élargi dans une direction (Z) orthogonale à la direction principale de mise en éventail (X) de telle sorte que l'élargissement correspond à la capacité de résolution (ε) de l'unité de détection dans cette direction et/ou dans lequel l'appareil d'imagerie (1) est un tomodensitomètre (1) qui est configuré pour émettre un rayon X en forme d'éventail (11) et pour détecter celui-ci avec un détecteur linéaire.
